## Europäisches Patentamt
## European Patent Office
## Office européen des brevets

(19)

(11) Numéro de publication: **0 055 970 B1**

(12) # FASCICULE DE BREVET EUROPEEN

(45) Date de publication du fascicule du brevet:
01.08.84

(51) Int. Cl.³: **C 07 C 53/12**, C 07 C 51/56

(21) Numéro de dépôt: 81420162.0

(22) Date de dépôt: 30.10.81

(54) **Procédé de préparation de l'anhydride acétique.**

(30) Priorité: **24.12.80 FR 8027941**

(73) Titulaire: **RHONE-POULENC CHIMIE DE BASE, 25, quai Paul Doumer, F-92408 Courbevoie (FR)**

(43) Date de publication de la demande:
**14.07.82 Bulletin 82/28**

(72) Inventeur: **Gauthier-Lafaye, Jean, 21, rue Duguesclin, F-69006 Lyon (FR)**
Inventeur: **Perron, Robert, La Pécolière, F-69390 Charly (FR)**

(45) Mention de la délivrance du brevet:
**01.08.84 Bulletin 84/31**

(74) Mandataire: **Varniere-Grange, Monique et al, RHONE-POULENC RECHERCHES Service Brevets Chimie et Polymères Centre de Recherches de Saint-Fons B.P. 62, F-69192 St-Fons Cédex (FR)**

(84) Etats contractants désignés:
**AT BE CH DE FR GB IT LI LU NL SE**

(56) Documents cités:
**FR - A - 2 438 023**
**FR - A - 2 439 764**
**GB - A - 2 007 666**
**US - A - 2 729 651**
**US - A - 4 002 678**
**US - A - 4 234 718**
**US - A - 4 234 719**

ACTORUM AG

## Description

La présente invention a pour objet un procédé de préparation de l'anhydride acétique par carbonylation de l'acétate de méthyle.

Il est bien connu que l'anhydride acétique peut être produit par carbonylation de l'acétate de méthyle, dans des conditions relativement sévères de pression en présence de complexes du nickel de formule

$$[A_4M]_2NiX_4$$

dans laquelle X représente un atome de brome ou d'iode, M un atome de phosphore ou d'azote, A étant, par exemple, un radical alkyle inférieur (cf. brevet des Etats-Unis d'Amérique No 2729651). Ces complexes, obtenus par réaction d'halogénures de nickel et d'halogénures de phosphonium ou d'ammonium quaternaire, peuvent être engagés sous cette forme dans la réaction en cause, ou bien ils peuvent être formés *in situ*. Toutefois, l'efficacité de ce type de procédé est faible malgré les hautes pressions mises en œuvre.

Des travaux plus récents (cf. la demande de brevet britannique No 2007666) ont montré que des conditions sensiblement plus douces peuvent être mises en œuvre lors de la carbonylation de l'acétate de méthyle en présence de nickel, d'iode (ou d'un composé de l'iode) et d'un promoteur choisi parmi les composés organiques des éléments trivalents du groupe de l'azote; des quantités non négligeables d'anhydride acétique peuvent alors être obtenues sous réserve que les deux conditions ci-après soient simultanément remplies:

— l'iode (ou le composé de l'iode) doit être engagé dans une proportion telle que la fraction d'iode qui n'est chimiquement liée ni avec le nickel ni avec le promoteur soit d'au moins 0,2 mol (en iode élémentaire) à la fois par mole de composé du nickel et par mole de promoteur;

— la réaction en cause doit être effectuée dans un acide carboxylique aliphatique comme solvant.

De l'examen des techniques antérieurement proposées, il ressort qu'il serait souhaitable de pouvoir produire efficacement l'anhydride acétique par carbonylation de l'acétate de méthyle en présence d'un système catalytique à base de nickel sans faire appel à des solvants organiques étrangers au milieu réactionnel, tout en opérant dans des conditions de pression relativement douces. Il serait également très souhaitable d'améliorer la productivité du système catalytique à base de nickel.

Il a maintenant été trouvé qu'il est possible de préparer efficacement l'anhydride acétique par carbonylation de l'acétate de méthyle en présence d'un système catalytique à base de nickel, en l'absence de solvants étrangers au milieu réactionnel, ledit milieu ne renfermant alors que la matière de départ, le système catalytique et, le cas échéant, des quantités plus ou moins importantes du produit de la réaction.

La présente invention a donc pour objet un procédé de préparation de l'anhydride acétique par carbonylation de l'acétate de méthyle en milieu anhydre, en phase liquide, en présence d'une quantité efficace de nickel, d'iodure de méthyle, d'un iodure d'ammonium quaternaire ou de phosphonium quaternaire et d'un sel de lithium, le milieu réactionnel renfermant, initialement, le cas échéant, de l'anhydride acétique.

Le procédé selon l'invention nécessite la présence d'une quantité efficace de nickel. N'importe quelle source de nickel peut être mise en œuvre dans le cadre du présent procédé. On peut introduire le nickel sous sa forme métallique (nickel Raney, par exemple) ou sous toute autre forme commode. A titre d'exemples de composés du nickel susceptibles d'être utilisés pour la mise en œuvre du présent procédé, on peut citer: le carbonate, l'oxyde, l'hydroxyde, les halogénures, en particulier l'iodure, les carboxylates, en particulier l'acétate, de nickel.

Néanmoins, lorsqu'on charge un sel de nickel, on peut observer une période d'induction plus ou moins longue et, de ce fait, on peut être amené à préférer l'emploi de composés de nickel zéro tel que le nickel tétracarbonyle et le bis(triphénylphosphine)nickel dicarbonyle. Bien entendu, les spécialistes seront à même de déterminer les formes appropriées des composés du nickel et ils verront naturellement que la forme précise sous laquelle le nickel est introduit dans le milieu réactionnel n'est pas fondamentale, en particulier dans le cadre d'un procédé continu.

La quantité de nickel n'est pas critique. La teneur en nickel qui a une influence sur la vitesse de réaction est déterminée en fonction de la vitesse de réaction que l'on estime convenable, compte tenu des autres paramètres de la réaction. De manière générale, une quantité comprise entre 5 et 2000 milliatomes-grammes de nickel par litre de solution conduit à des résultats satisfaisants. On opère de préférence avec une teneur comprise entre 20 et 1000 milliatomes-grammes de nickel par litre.

La mise en œuvre de la présente invention nécessite également la présence dans le milieu réactionnel d'iodure de méthyle. Il n'est pas nécessaire de charger au départ ce composant du système catalytique et on peut faire appel, par exemple, à de l'iode libre, à de l'acide iodhydrique, à un iodure d'alkyle, différent de l'iodure de méthyle, ou à un iodure d'acyle. Comme les spécialistes le savent, l'iode et ces types de composés iodés peuvent être considérés comme des précurseurs de l'iodure de méthyle dans la réaction en cause.

En général, l'iodure de méthyle est présent dans le milieu réactionnel à raison de 1 à 100 mol et, de préférence, à raison de 5 à 50 mol par atome-gramme de nickel se trouvant dans ledit milieu.

Le système catalytique mis en œuvre dans le cadre du présent procédé compend également un iodure d'ammonium quaternaire ou de phosphonium quaternaire.

La nature précise de ces iodures n'est pas fondamentale et le choix parmi ces composés est davantage orienté par des considérations d'ordre pratique telles que la disponibilité, la commodité d'emploi et la solubilité dans le milieu réactionnel, milieu qui, comme cela a déjà été précisé, est exempt de solvant organique étranger.

A ce titre, la titulaire préconise l'utilisation d'iodures d'ammonium ou de phosphonium quaternaire dont les cations sont représentés respectivement par les formules (I) et (II) ci-après:

$$R_1N^+(R_2)_3 \qquad (I)$$

$$R_1P^+(R_2)_3 \qquad (II)$$

dans lesquelles $R_1$ et $R_2$, identiques ou différents, représentent des radicaux alkyles linéaires ayant au maximum 4 atomes de carbone, $R_2$ pouvant en outre représenter un radical phényle, tolyle ou xylyle.

A titre d'exemples d'iodures d'ammonium quaternaire, convenant à la mise en œuvre du présent procédé, on peut citer les iodures de tétraméthylammonium, triéthylméthylammonium, tributylméthylammonium, tributyl(n-propyl)ammonium, tétraéthylammonium et de tétrabutylammonium.

A titre d'exemples d'iodures de phosphonium quaternaire convenant à la mise en œuvre du présent procédé, on peut citer les iodures de méthyltriphénylphosphonium, d'éthyltriphénylphosphonium, de méthyltrixylylphosphonium et de méthyltributylphosphonium.

Bien entendu, ce type de composés, dont la présence est nécessaire à la mise en œuvre de la présente invention, peut être formé *in situ* à partir de l'amine ou de la phosphine correspondante introduite, le cas échéant, sous la forme d'un complexe de nickel tel que le bis(triphénylphosphine)nickel dicarbonyle, et d'un iodure d'alkyle. Si on choisit ce mode opératoire, il conviendra de charger, outre l'amine (ou la phosphine) en cause, la quantité d'iodure d'alkyle (le cas échéant, d'iodure de méthyle) nécessaire à sa quaternisation de telle sorte que cette transformation *in situ* ne se produise pas au détriment de l'iodure de méthyle dont la présence dans le milieu réactionnel est également nécessaire.

On peut faire usage de mélanges de tels composés. En général, ces composés sont présents dans le milieu réactionnel en quantité telle que le rapport atomique ou phosphore et/ou de l'azote au nickel soit compris entre 0,2 et 50 et, de préférence, entre 0,2 et 20.

De manière avantageuse, ce rapport est compris entre 0,5 et 10. Les iodure de phosphonium quaternaire conviennent plus particulièrement bien à la mise en œuvre du présent procédé.

Selon le procédé, objet de la présente invention, il est essentiel que le système catalytique renferme un sel de lithium. La nature précise de l'anion de ce sel n'est pas fondamentale et, à titre d'exemples de sels de lithium utilisables dans le cadre du présent procédé, on peut citer: l'hydroxyde, le chlorure, le bromure, l'iodure, le carbonate et le nitrate de lithium ainsi que les carboxylates de lithium renfermant au maximum 12 atomes de carbone.

Parmi ces sels, l'iodure, le carbonate et les carboxylates de lithium conviennent particulièrement bien à la mise en œuvre de la présente invention. On utilise, de préférence, un carboxylate de lithium ayant au maximum 5 atomes de carbone, l'acétate de lithium se montrant particulièrement efficace.

En général, on utilise un (ou plusieurs) sel(s) de lithium en quantité telle que le rapport atomique du lithium au nickel soit compris entre 1 et 100, bien que des quantités inférieures ou supérieures puissent être mises en jeu. De bons résultats sont obtenus pour un rapport atomique Li/Ni compris entre 2 et 25.

Le système catalytique défini ci-avant se révèle particulièrement efficace pour préparer l'anhydride acétique par carbonylation de l'acétate de méthyle, en phase liquide, sans faire appel à des solvants étrangers au milieu réactionnel.

Il a été également trouvé, de manière tout à fait inattendue, que l'anhydride acétique (produit de la réaction) non seulement n'inhibe par la réaction de carbonylation en présence du système catalytique en cause, mais tend à favoriser le bon déroulement de celle-ci.

Ainsi, dans le cadre d'un mode d'exécution avantageux du présent procédé, on opérera sur un mélange d'acétate de méthyle et d'anhydride acétique dans lequel l'anhydride acétique représentera de l'ordre de 10 à 90% en volume et, de préférence, de l'ordre de 20 à 80% dudit volume.

Lorsque la réaction sera conduite en discontinu, on aura tout avantage à introduire dès le départ la quantité souhaitée d'anhydride acétique et, lorsque la réaction sera exécutée en continu, il conviendra de régler le débit des diverses matières à l'entrée et à la sortie de la zone réactionnelle de telle sorte que la quantité voulue d'anhydride acétique soit présente dans la zone réactionnelle. Dans ce dernier cas, il peut être utile de recycler une partie de l'anhydride acétique produit, dans la zone de réaction.

Comme indiqué en tête du présent mémoire, la réaction est conduite en phase liquide sous une pression supérieure à la pression atmosphérique. En général, on opère sous une pression totale supérieure à 15 bar; il n'est pas utile d'atteindre 700 bar. Pour une bonne mise en œuvre de l'invention, une pression totale de 25 à 200 bar est préconisée.

La température de réaction est généralement supérieure à 140° C, sans qu'il soit nécessaire d'atteindre 300° C. De bons résultats sont obtenus dans la gamme de températures allant de 160 à 220° C.

On met en œuvre, de préférence, du monoxyde de carbone sous forme essentiellement pure, tel qu'il se présente dans le commerce. Toutefois, la présence d'impuretés telles que du dioxyde de carbone, de l'oxygène, du méthane et de l'azote peut être tolérée. La présence d'hydrogène n'est pas nuisible, même en des proportions relativement importantes.

En fin d'opération, l'anhydride acétique obtenu est séparé des autres constituants du milieu réactionnel par tout moyen approprié, par exemple par distillation.

Les exemples ci-après illustrent l'invention sans toutefois en limiter le domaine ou l'esprit.

Les conventions suivantes sont utilisées ci-après:

RR (%) désigne le nombre de moles d'anhydride acétique produites pour 100 mol d'acétate de méthyle chargées.

Pr désigne la productivité en grammes d'anhydride acétique par heure et par litre du milieu réactionnel initial.

*Exemple 1:*

Dans un autoclave en Hastelloy B2 et de 125 ml de capacité, on introduit:
— 25 ml d'acétate de méthyle,
— 20 ml d'anhydride acétique,
— 80 mmol d'iodure de méthyle,
— 4 milliatomes-grammes de nickel sous forme de bis(triphénylphosphine)nickel dicarbonyle,
— 12 mmol d'iodure de méthyltriphénylphosphonium, et
— 100 mmol d'acétate de lithium.

Après fermeture de l'autoclave, on établit une pression de 40 bar de monoxyde de carbone. L'agitation par un système de va-et-vient est mise en route et l'autoclave est porté à 180° C, en 25 min environ, au moyen d'un four annulaire. La pression dans l'autoclave est maintenue constante et égale à 70 bar par alimentation continue de monoxyde de carbone. Après 2 h de réaction à la température indiquée, les résultats obtenus sont les suivants:
RR (%) = 60
Pr = 190

*Essai témoin (a):*

On reproduit l'exemple 1 ci-avant en l'absence d'acétate de lithium. Les résultats obtenus sont les suivants:
RR (%) = 15
Pr = 48

La comparaison entre ces résultats et ceux obtenus dans l'exemple 1 montre que la présence d'un sel de lithium permet de multiplier la quantité d'anhydride acétique par un facteur de l'ordre de 4.

*Exemple 2:*

Dans un autoclave et selon le mode opératoire décrits ci-dessus, on réalise un essai sur une charge constituée de:
— 25 ml d'acétate de méthyle,
— 20 ml d'anhydride acétique,
— 90 mmol d'iodure de méthyle,
— 4 milliatomes-grammes de nickel sous forme de bis(triphénylphosphine)nickel dicarbonyle,
— 12 mmol d'iodure de méthyltriphénylphosphonium, et
— 50 mmol de carbonate de lithium.

Les résultats obtenus en 2 h de réaction à 180° C et sous une pression totale de 90 bar sont les suivants:
RR (%) = 56
Pr = 175

*Exemple 3:*

On reproduit l'exemple 2 ci-dessus en remplaçant le carbonate de lithium par 40 mmol d'iodure de lithium et en ne chargeant que 79 mmol d'iodure de méthyle. Les résultats sont les suivants:
RR (%) = 46
Pr = 145

*Exemple 4:*

Dans un autoclave et selon le mode opératoire décrits ci-dessus, on réalise un essai sur une charge constituée de:
— 25 ml d'acétate de méthyle,
— 20 ml d'anhydride acétique,
— 96 mmol d'iodure de méthyle,
— 4 milliatomes-grammes de nickel sous forme de bis(triphénylphosphine)nickel dicarbonyle,
— 12 mmol d'iodure de méthyltriphénylphosphonium, et
— 40 mmol d'acétate de lithium.

Les résultats obtenus en 2 h de réaction à 180° C et sous une pression totale de 70 bar comprenant 20 bar de pression partielle d'hydrogène sont les suivants:
RR (%) = 45
Pr = 145

*Exemple 5:*

Dans un autoclave et selon le mode opératoire décrits ci-dessus, on réalise un essai sur une charge constituée de:
— 25 ml d'acétate de méthyle,
— 20 ml d'anhydride acétique,
— 80 mmol d'iodure de méthyle,
— 4 milliatomes-grammes de nickel sous forme de nickel tétracarbonyle,
— 20 mmol d'iodure de méthyltriphénylphosphonium, et
— 40 mmol d'acétate de lithium.

Les résultats obtenus en 2 h de réaction à 180° C et sous une pression totale de 90 bar sont les suivants:
RR (%) = 63
Pr = 200

*Exemple 6:*

On reproduit l'exemple 5 ci-dessus en divisant par 5 la quantité d'iodure de méthyltriphénylphosphonium. Les résultats sont les suivants:
RR (%) = 66
Pr = 210

*Essai témoin (b):*

On reproduit l'exemple 5 ci-dessus en l'absence d'iodure de méthyltriphénylphosphonium, la durée de réaction n'étant que de 1½ h. Les résultats obtenus sont les suivants:
RR (%) = 6
Pr = 25

*Exemples 7 à 13:*

Dans l'autoclave, et selon le mode opératoire décrits pour l'exemple 1, on a réalisé une série d'essais sur une charge constituée de 25 ml d'acétate de méthyle, 20 ml d'anhydride acétique, 40 mmol d'acétate de lithium, d'iodure de méthyle, de bis(triphénylphosphine)nickel dicarbonyle et, le cas échéant, d'iodure de méthyltriphénylphosphonium. Les conditions particulières ainsi que les résultats obtenus en 2 h de réaction à 180° C sont rassemblés dans le tableau ci-après, dans lequel $P_T$ désigne la pression totale et $P^+I^-$ (mmol) la quantité d'iodure de méthyltriphényl-phosphonium chargée.

| No | Nickel (mAt-g) | CH$_3$ I (mmol) | P$^+$ I$^-$ (mmol) | P$_T$ (bar) | RR (%) | Pr |
|---|---|---|---|---|---|---|
| 7 | 4 | 35 | 12 | 70 | 36 | 115 |
| 8 | 2 | 85 | 16 | 90 | 40,5 | 125 |
| 9 | 4 | 88 | 12 | 70 | 54 | 170 |
| 10 | 8 | 95 | 4 | 70 | 59 | 190 |
| 11 | 4 | 160 | 12 | 70 | 62 | 195 |
| 12 | 4 | 89 | 11 | 90 | 65 | 210 |
| 13 | 4 | 88 | 0 | 90 | 71 | 225 |

*Exemple 14:*

Dans l'autoclave et selon le mode opératoire décrits pour l'exemple 1, on réalise un essai sur une charge constituée de:
 — 45 ml d'acétate de méthyle,
 — 80 mmol d'iodure de méthyle,
 — 4 milliatomes-grammes de nickel sous forme de bis(triphénylphosphine)nickel dicarbonyle,
 — 12 mmol d'iodure de méthyltriphénylphosphonium, et
 — 19,8 mmol d'acétate de lithium.
 Les résultats obtenus en 2 h de réaction à 180° C et sous une pression totale de 70 bar sont les suivants:
 RR (%) = 19
 Pr = 110

**Revendications**

1. Procédé de préparation de l'anhydride acétique par carbonylation de l'acétate de méthyle en milieu anhydre, en phase liquide, en présence d'une quantité efficace de nickel, d'iodure de méthyle, d'un iodure d'ammonium quaternaire ou de phosphonium quaternaire et d'un sel de lithium, le milieu réactionnel renfermant, initialement, le cas échéant, de l'anhydride acétique.

2. Procédé selon la revendication 1, caractérisé en ce que la pression totale est comprise entre 15 et 700 bar et, de préférence, entre 25 et 200 bar.

3. Procédé selon l'une des revendications 1 ou 2, caractérisé en ce que la température est comprise entre 140 et 300° C, de préférence, entre 160 et 220° C.

4. Procédé selon l'une des revendications précédentes, caractérisé en ce que la concentration du nickel est comprise entre 5 et 2000 milliatomes-grammes par litre de milieu réactionnel.

5. Procédé selon la revendication 4, caractérisé en ce que la concentration du nickel est comprise entre 20 et 1000 milliatomes-grammes par litre de milieu réactionnel.

6. Procédé selon l'une des revendications précédentes, caractérisé en ce que l'iodure de méthyle est présent dans le milieu réactionnel à raison de 1 à 100 mol par atome-gramme de nickel.

7. Procédé selon la revendication 6, caractérisé en ce que l'iodure de méthyle est présent dans le milieu réactionnel à raison de 5 à 50 mol par atome-gramme de nickel.

8. Procédé selon l'une des revendications précédentes, caractérisé en ce qu'on opère en présence d'un iodure de phosphonium quaternaire.

9. Procédé selon l'une des revendications précédentes, caractérisé en ce que le rapport atomique du phosphore et/ou de l'azote au nickel est compris entre 0,2 et 50 et, de préférence, entre 0,2 et 20.

10. Procédé selon l'une des revendications précédentes, caractérisé en ce que le sel de lithium est un carboxylate ayant au maximum 12 atomes de carbone.

11. Procédé selon l'une des revendications précédentes, caractérisé en ce que le sel de lithium est l'acétate de lithium.

12. Procédé selon l'une des revendications précédentes, caractérisé en ce que le rapport atomique du lithium au nickel est compris entre 1 et 100.

13. Procédé selon l'une des revendications précédentes, caractérisé en ce que le rapport atomique du lithium au nickel est compris entre 2 et 25.

14. Procédé selon l'une des revendications précédentes, caractérisé en ce qu'on opère sur un mélange d'acétate de méthyle et d'anhydride acétique, l'anhydride acétique représentant 10 à 90% et, de préférence, de 20 à 80% du volume de ce mélange.

**Patentansprüche**

1. Verfahren zur Herstellung von Essigsäureanhydrid durch Carbonylieren von Methylacetat in wasserfreiem Medium, in flüssiger Phase, in Gegenwart einer wirksamen Menge Nickel, Methyljodid, eines quaternären Ammonium- oder Phosphoniumjodids und eines Lithiumsalzes, wobei das Reaktionsmedium zu Beginn gegebenenfalls Essigsäureanhydrid enthält.

2. Verfahren nach Anspruch 1, dadurch gekennzeichnet, dass der Gesamtdruck 15 bis 700, vorzugsweise 25 bis 200 bar, beträgt.

3. Verfahren nach einem der beiden Ansprüche 1 oder 2, dadurch gekennzeichnet, dass die Temperatur 140 bis 300, vorzugsweise 160 bis 220° C, beträgt.

4. Verfahren nach einem der vorangehenden Ansprüche, dadurch gekennzeichnet, dass die Nickelkonzentration 5 bis 2000 mG-Atom je Liter Reaktionsmedium ausmacht.

5. Verfahren nach Anspruch 4, dadurch gekennzeichnet, dass die Nickelkonzentration 20 bis 100 mG-Atom je Liter Reaktionsmedium ausmacht.

6. Verfahren nach einem der vorangehenden Ansprüche, dadurch gekennzeichnt, dass das Methyljodid im Reaktionsmedium in einer Menge von 1 bis 100 mol je G-Atom Nickel vorhanden ist.

7. Verfahren nach Anspruch 6, dadurch gekennzeichnet, dass das Methyljolid im Reaktionsmedium in einer Menge von 5 bis 50 mol je G-Atom Nickel enthalten ist.

8. Verfahren nach einem der vorangehenden Ansprüche, dadurch gekennzeichnet, dass man in Gegenwart eines quaternären Phosphoniumjodids arbeitet.

9. Verfahren nach einem der vorangehenden Ansprüche, dadurch gekennzeichnet, dass das Atomverhältnis von Phosphor und/oder Stickstoff zu Nickel 0,2 bis 50, vorzugsweise 0,2 bis 20, beträgt.

10. Verfahren nach einem der vorangehenden Ansprüche, dadurch gekennzeichnet, dass das Lithiumsalz das Salz einer Carbonsäure mit maximal 12 Kohlenstoffatomen ist.

11. Verfahren nach einem der vorangehenden Ansprüche, dadurch gekennzeichnet, dass das Lithiumsalz Lithiumacetat ist.

12. Verfahren nach einem der vorangehenden Ansprüche, dadurch gekennzeichnet, dass das Atomverhältnis von Lithium zu Nickel 1 bis 100 beträgt.

13. Verfahren nach einem der vorangehenden Ansprüche, dadurch gekennzeichnet, dass das Atomverhältnis von Lithium zu Nickel 2 bis 25 beträgt.

14. Verfahren nach einem der vorangehenden Ansprüche, dadurch gekennzeichnet, dass man mit einem Gemisch aus Methylacetat und Essigsäureanhydrid arbeitet, wobei das Essigsäureanhydrid 10 bis 90, vorzugsweise 20 bis 80 Vol.-%, des Gemisches ausmacht.

## Claims

1. Process for the preparation of acetic anhydride by the carbonylation of methyl acetate in an anhydrous medium, in the liquid phase, in the presence of an effective amount of nickel, methyl iodide, a quaternary ammonium or quaternary phosphonium iodide and a lithium salt, the reaction mixture optionally initially containing acetic anhydride.

2. Process according to Claim 1, characterised in that the total pressure is between 15 and 700 bar and preferably between 25 and 200 bar.

3. Process according to Claim 1 or 2, characterised in that the temperature is between 140 and 300° C and preferably between 160 and 220° C.

4. Process according to any one of the preceding claims, characterised in that the concentration of the nickel is between 5 and 2,000 milligrams atoms per litre of reaction medium.

5. Process according to Claim 4, characterised in that the concentration of the nickel is between 20 and 1,000 milligrams atoms per litre of reaction medium.

6. Process according to any one of the preceding claims, characterised in that the methyl iodide is present in the reaction medium in an amount of 1 to 100 mol per gram atom of nickel.

7. Process according to Claim 6, characterised in that the methyl iodide is present in the reaction medium in an amount of 5 to 50 mol per gram atom of nickel.

8. Process according to any one of the preceding claims, characterised in that the reaction is carried out in the presence of a quaternary phosphonium iodide.

9. Process according to any one of the preceding claims, characterised in that the atomic ratio of the phosphorus and/or the nitrogen to the nickel is between 0.2 and 50 and preferably between 0.2 and 20.

10. Process according to any one of the preceding claims, characterised in that the lithium salt is a carboxylate having at most 12 carbon atoms.

11. Process according to any one of the preceding claims, characterised in that the lithium salt is lithium acetate.

12. Process according to any one of the preceding claims, characterised in that the atomic ratio of the lithium to the nickel is between 1 and 100.

13. Process according to any one of the preceding claims, characterised in that the atomic ratio of the lithium to the nickel is between 2 and 25.

14. Process according to any one of the preceding claims, characterised in that the reaction is carried out on a mixture of methyl acetate and acetic anhydride, the acetic anhydride representing 10 to 90% and preferably from 20 to 80% of the volume of this mixture.